Europäisches Patentamt

European Patent Office

Office européen des brevets

(11) Publication number: **0 309 961**
**A1**

## EUROPEAN PATENT APPLICATION

(21) Application number: 88115803.4

(22) Date of filing: 26.09.88

(51) Int. Cl.4: **C12N 15/00 , C07H 21/04 , C12N 1/20 , //(C12N1/20, C12R1:19)**

Claims for the following Contracting State: ES.

(30) Priority: 29.09.87 JP 245042/87

(43) Date of publication of application:
05.04.89 Bulletin 89/14

(84) Designated Contracting States:
AT BE CH DE ES FR GB GR IT LI LU NL SE

(71) Applicant: HOECHST JAPAN LIMITED
10-16, 8-chome, Akasaka, Minato-ku
Tokyo(JP)

(72) Inventor: Toba, Mari
1-37-23, Kishi-machi
Kawagoe-shi Saitama-ken(JP)
Inventor: Hashimoto, Tamotsu
1-5-10-309, Sakoe-cho
Asaka-shi Saitama-ken(JP)

(74) Representative: Klein, Otto, Dr. et al
Hoechst AG Zentrale Patentabteilung
Postfach 80 03 20
D-6230 Frankfurt am Main 80(DE)

(54) Plasmid vector containing streptomycin-sensitivity gene.

(57) A DNA segment containing a streptomycin-sensitivity gene with a nonsense codon or nonsense codons in the coding region is used to obtain a plasmid vector containing the streptomycin-sensitivity gene having multiple cloning sites, which gene can be switched on and off for expression depending on the medium conditions and the type of host bacteria. From this streptomycin-sensitive plasmid vector recombinant molecules can thus be directly selected.

EP 0 309 961 A1

# PLASMID VECTOR CONTAINING STREPTOMYCIN-SENSITIVITY GENE

## BACKGROUND OF THE INVENTION

The present invention relates to a gene coding for streptomycin-sensitivity (hereinafter referred to as a streptomycin-sensitivity gene $strA^S$ or $Sm^S$) having multiple cloning sites and to a recombinant plasmid vector containing this gene whose expression can be controlled by the selection of host bacteria and, optionally, of medium conditions.

In gene manipulation, an effective technique for cloning an entire desired gene or for subcloning a part of the gene is important. In such cloning experiments, one has to consider various characteristics of plasmid vectors used, such as plasmid size, plasmid copy number per cell, available cloning sites, drug resistance marker for selection and dependency on the genomic characteristics of host cells, which may affect the efficiency and success of an experiment.

With respect to such features of plasmid vectors, it is desirable to establish a culture condition to allow only cells containing a recombinant molecule to grow selectively in order to effectively and exclusively select a vector with insertion of a foreign DNA fragment (hereinafter referred to as a recombinant molecule) in a medium. Although a number of plasmid vectors for this purpose have been developed and reported, they have not been frequently used because the number of available cloning sites are limited and/or because the culture conditions are excessively specific or complicated. For this purpose, we have previously developed plasmid vectors, pHSG666 and pHSG670, with which one can grow selectively host cells harboring recombinant molecules by simply adding streptomycin to a medium. These vectors were disclosed in the patent applications by us (Japanese Patent Applications Nos. 60-253192 and 61-190798 or EP-A 0,223,156).

When introduced into ampicillin sensitive host cells having a streptomycin-resistance gene ($strA^R$), these vectors dominantly express ampicillin resistance and streptomycin sensitivity owing to their ampicillin-resistance gene ($Ap^R$) and the streptomycin-sensitivity gene ($strA^S$ or $rpsL^+$). However, in the case where foreign DNA fragments are inserted in the gene ($rpsL^+$), by "inactivation cloning", or said gene is altered or deleted, the streptomycin sensitivity is not dominantly expressed, and consequently the host cell can grow in the presence of streptomycin and ampicillin.

As plasmid vectors which allow only bacteria having a recombinant molecule to grow selectively in the presence of streptomycin, we have previously developed pHSG664 [Gene, 41, 125-128 (1986)], pHSG666 and pHSG670 (Japanese Patent Applications Nos. 60-253192 and 61-190798 or EP-A 0,223,156). Among them, pHSG666 has been deposited with the Fermentation Research Institute, Agency of Industrial Science & Technology, Japan, under the deposition number FERM BP-1183 (FERM P-8902). Furthermore, pHSG670 can be easily constructed from pHSG666 as described in Japanese Patent Applications Nos. 60-253192 and 60-190798 or in EP-A 0,223,156.

However, among these plasmid vectors, it now appears that pHSG670 particularly undergoes spontaneous alteration or degradation of the streptomycin-sensitivity gene in growing cells, and such changes are so frequent that the resultant molecules can no longer transform their streptomycin-resistant host cells into streptomycin-sensitive ones. Thus cells containing non-recombinant molecules can grow in a medium containing streptomycin as substantial background cells and can no longer be distinguished from transformed cells which are streptomycin-resistant due to inactivation cloning. This effect is probably due to an overproduction of the ribosomal small subunit S12 protein, one of the components of the ribosome complex, which is a product of the streptomycin-sensitivity gene, which interferes with the protein synthesis system in host cells and consequently disturbs the growth of the host cells so as to lead to an overgrowth of the cells carrying plasmid molecules with a degraded streptomycin gene.

Under these circumstances, an improvement of a streptomycin-sensitivity gene to be expressed only during the recombinant experiment but not during the bacterial cell culture for the vector DNA preparation is desired.

## SUMMARY OF THE INVENTION

We have accomplished the construction of specific vectors (named pHSG673 and pHSG683), streptomycin-sensitive plasmids from which recombinant molecules can be directly selected and whose streptomycin sensitivity gene has not only numerous cloning sites but also can be switched on and off for expression depending on the medium conditions and the type of host bacteria, and we have further found that almost no non-recombinant molecules appear in the background during cloning experiments, and thus we have achieved the present invention as described below.

The DNA segment according to the present invention contains a streptomycin-sensitivity gene having at least one nonsense codon in an essential area of the coding region. Thus, in wild-type host cells this gene is not or not effectively expressed. However, in suppressor mutant cells there occurs an effective expression of the sensitivity gene.

In preferred embodiment the DNA segment contains an inducible promoter, e.g. the tryptophan promoter, upstream of the streptomycin-sensitivity gene so as to control the expression of said gene.

The present invention also relates to a plasmid vector containing this DNA segment.

Especially, the plasmid vector according to the present invention is selected from the group consisting of plasmid vectors named pHSG673 and pHSG683, each of which has a DNA segment containing a streptomycin-sensitivity gene with a nonsense codon in the coding region and operatively linked to an inducible promoter, e.g. the tryptophan promoter so as to control the expression of said gene downstream thereof.

Furthermore, the present invention also relates to a microorganism carrying the aforementioned plasmid vector.

The DNA segment according to the present invention and particularly the modified type of a streptomycin-sensitivity plasmid vector which have features (a) to (e) as described below and whose advantages have been confirmed in the experimental examples described hereinafter provide a means for a highly efficient cloning and subcloning in a recombinant DNA experiment.

Table 1 illustrates the nucleotide and amino acid sequences of the region of the streptomycin-sensitivity gene contained in pHSG673 and pHSG683.

## BRIEF DESCRIPTION OF THE DRAWINGS

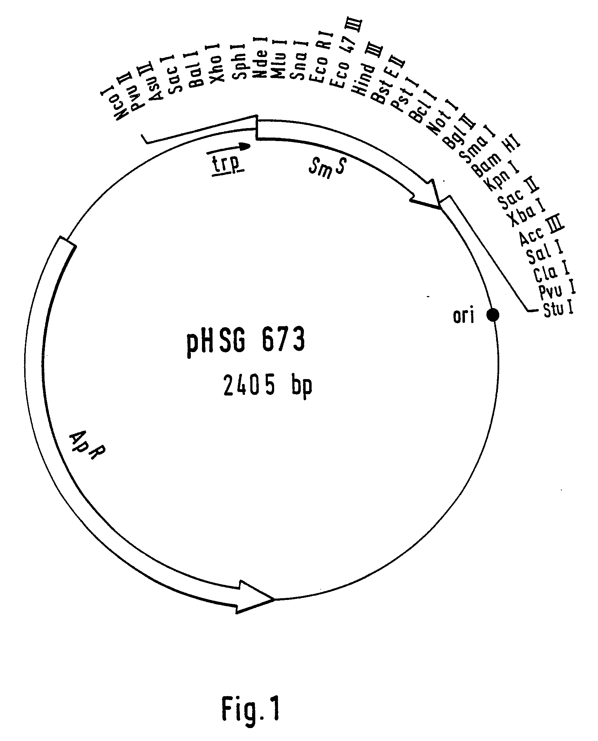

FIG. 1 is a restriction enzyme cleavage map for pHSG673.
FIG. 2 is a restriction enzyme cleavage map for pHSG683.

In these drawings "trp" means the trp promoter, "Sm$^S$" means the streptomycin-sensitivity gene, "Cm$^R$" means the chloramphenicol-resistance gene and "Ap$^R$" means the ampicillin-resistance gene.

These figures are not drawn to scale.

## DETAILED DESCRIPTION OF THE INVENTION

The DNA segment of the present invention

The DNA segment of the present invention contains a streptomycin-sensitivity coding region, which in turn is characterized by having a nonsense codon in an essential area.

The term "contains" as used herein means that the DNA segment may also include other genes or DNA segments besides this streptomycin-sensitivity coding region.

An example of other DNA segments than the streptomycin-sensitivity coding region is an inducible promoter which is located upstream of the streptomycin-sensitivity coding region so as to control the expression thereof.

Accordingly, a representative DNA segment according to the present invention is one, as aforementioned, that contains a streptomycin-sensitivity coding region and a promoter upstream thereof.

Examples of promoters capable of expressing a streptomycin-sensitivity gene include but are not limited to the lac promoter, tac promoter and trp promoter.

Furthermore, the nonsense (or stop) codons are the amber codon TAG, the ochre codon TAA and the opal codon TGA.

Suppressor cells having abnormal transfer RNA (tRNA) molecules are well-known (A. Garen, Science

3

160 (1968) 149-159). These tRNA molecules are coded for by suppressor genes. The E. coli genome contains several suppressor genes: sup D, E and F code for tRNAs which recognize the amber codon, and sup C and G which either recognize the amber or the ochre codon, and which, therefore, do not lead to a stop of the protein synthesis but rather build in an amino acid. Thus the sup F codes for a tyrosine tRNA which "reads" the amber stop codon for a tyrosine codon.

The requirement that the nonsense codon be in an "essential area means that its expression in wild-type host cells is either prohibited or it leads to an inactive molecule. Accordingly, introduction of a nonsense codon near the end of the gene still may result in an expression of an active enzyme in wild-type host cells - then the nonsense codon would not be located in an "essential" area.

Accordingly, a representative embodiment of the DNA segment according to the present invention has the nucleotide sequence shown in Table 1, in which the nonsense codon in the coding region is the amber codon and the inducible promoter is the tryptophan promoter.

One of the embodiments of the DNA segment according to the present invention is that in the form of a plasmid vector.

Representative plasmid vectors according to the present invention are those named pHSG673 and pHSG683, each containing a DNA segment as described above as an embodiment of the present invention, i. e., a DNA segment having a streptomycin-sensitivity coding segment driven by an inducible promoter upstream thereof.

The DNA segment or plasmid vector according to the present invention, particularly the aforementioned embodiments thereof, have the following useful features:

(1) the expression of the streptomycin-sensitivity gene can be regulated, e.g. by the tryptophan concentration in the medium when said gene is under the control of the tryptophan promoter;

(2) no S12 protein whatsoever is produced from the plasmid vectors in a wild type cell lacking any suppressor mutation because the streptomycin-sensitivity gene has the amber mutation;

(3) owing to the features as described in (1) and (2) above, in the case where wild type cells containing pHSG673 or pHSG683 are grown in a medium containing tryptophan, there is no growth inhibitory effect on the host cells so that molecules with degraded streptomycin-sensitivity genes will not be accumulated in the plasmid population;

(4) accordingly, in the case where a cloning experiment is carried out with pHSG673 or pHSG683, if, after the vector DNA preparation, a suppressor mutant strain (supE⁻) is used as a host and selected in the medium deprived of tryptophan, almost no background colonies due to non-recombinant molecules lacking in foreign DNA fragments will appear; and

(5) as compared to pHSG670, PvuI and NdeI restriction sites are newly introduced as single cloning sites in the streptomycin-sensitivity coding region per vector molecule.

## Construction of plasmid vectors

Plasmid vectors pHSG673 and pHSG683 according to the present invention can be constructed by a method suitable for the purpose.

An example of such a method comprises the following steps (a) to (i):

(a) removing the PvuI site from the ampicillin-resistance gene by treating pHSG666 DNA with nitrosoguanidine;

(b) isolating and purifying from the plasmid DNA obtained in the step (a), a DNA fragment between the EcoRI site and the AsuII site containing most of the vector DNA except for the portion containing the promoter of the strA$^S$ gene;

(c) synthesizing and purifying a DNA fragment coding for the tryptophan promoter and for the amino terminal of the streptomycin-sensitivity gene between the EcoRI site and the AsuII site by means of the phosphoamidite method using, for example, a DNA synthesizer ( e. g., a product of Applied Biosystem Co., USA);

(d) ligating the DNA fragments obtained in the steps (b) and (c) to construct an ampicillin-resistance vector having a streptomycin-sensitivity gene whose expression is driven by the tryptophan promoter/operator;

(e) removing the EcoRI-SspI fragment upstream of the tryptophan promoter of the plasmid constructed in the step (d) by digesting with the corresponding enzymes followed by blunting the terminal ends with T4 DNA polymerase and religating with T4 DNA ligase, and then obtaining the vector DNA fragment flanked by XhoI and BglI in which the EcoRI-SspI region was deleted as described above;

(f) isolating and purifying a DNA fragment flanked by XhoI and BglI containing the replication region between the ampicillin-resistance gene and the streptomycin-sensitivity gene from plasmid pHSG670;

(g) ligating the DNA fragments obtained in the steps (e) and (f) to construct a plasmid vector (named pHSG672 in the present invention) having a replicon derived from pHSG666 and having, as selection markers, the ampicillin-resistance gene and the streptomycin-sensitivity gene, the latter of which is expressed by the tryptophan promoter;

(h) constructing a plasmid (named pHSG673 in the present invention) with a replacement in the plasmid pHSG672 constructed in the steps (g) in such a manner that a DNA fragment between the SphI site and the MluI site coding for from the 26th amino acid, alanine, to the 37th amino acid, valine, is replaced by a corresponding DNA fragment in which the 29th codon CAG for glutamine has been replaced by the amber codon TAG and the 33th codon for valine has been replaced by the isoleucine codon ATA, each of the position numbers being counted from the first methionine residue of the streptomycin-sensitivity gene; and

(i) constructing a plasmid vector (named pHSG683 in the present invention) in which the DNA fragment containing the ampicillin-resistance gene of pHSG673 plasmid constructed in the step (h) is replaced by a DNA fragment containing a chloramphenicol-resistance gene of, for example, pHSG396 commercially available from Takara Shuzo Co. Ltd., Japan.

As described above, the starting material, plasmid pHSG666, has been deposited at the Fermentation Research Institute, Agency of Industrial Science & Technology, Japan (FERM P-8902), and plasmid pHSG670 can be easily constructed from pHSG666.

For reference, a method for construction of pHSG670 is as follows.

The DNA of pHSG666 is digested with EcoRI, blunt ended by T4-DNA polymerase and religated with T4 DNA ligase. The DNA of this plasmid thus obtained is digested with HindIII, blunt ended, and religated to obtain a plasmid designated as p1634. The portion between the SphI site and the PstI site in the strA$^S$ gene of p1634 is replaced by the DNA linkers having the formulae shown below to obtain pHSG670.

Formulae:

5'-CCCGCAGAAGCGTGGTGTGTGCACACGCGTATACACTACTACTCCGAAGAAACCG-3'

3'-GTACGGGCGTCTTCGCACCACACACGTGTGCGCATATGTGATGATGAGGCTTCTTTGGCTTAA-5'

and

5'-AATTCAGCGCTGCGCAAGCTTTGCCGCGTACGCCTGACCAACGGTTTCGAG-3'

3'-GTCGCGACGCGTTCGAAACGGCGCATGCGGACTGGTTGCCAAAGCTCCAGTG-5'

and

5'-GTCACCTCATATATAGGTGGTGAAGGACACAACCTGCA-3'

3'-GAGTATATATCCACCACTTCCTGTGTTGG-5'

Host bacteria used for the preparation of a vector DNA are typically wild-type strains of Escherichia coli (E. coli) K12, preferably recA⁻ strain. Examples of these strains are F⁻Z⁻4M15 recA [Nucleic Acids Research, 9, 4087-4098 (1981)] and MC1009 [Journal of Molecular Biology, 138, 179-207 (1981); Gene, 15, 99-102 (1981)]. These strains are broadly used for recombinant DNA experiments and are thus readily obtainable.

As host bacteria for selection of transformed colonies with recombinant molecules, the HB101 strain can be used as was used for pHSG670 or pHSG671 in the aforementioned inventions of the previous applications.

Experimental examples

Example 1

Construction of the streptomycin-sensitivity gene controlled by the tryptophan promoter/operator

(1) Synthesis of the DNA coding for the tryptophan promoter/operator:

DNA linkers having the formulae:

5'-AATTCAATATTCTGAAATGAGCTGTTGACAATTAATCATCGAACTAGTTAACTA-3'

3'-GTTATAAGACTTTACTCGACAACTGTTAATTAGTAGCTTGATCAATTGATCATGCG-5'

and

5'-GTACGCAAGTTCACGTAAAAAGGGTATCGACCATGGCAACAGTTAACCAGCTGGTT-3'

3'-TTCAAGTGCATTTTTTCCCATAGCTGGTACCGTTGTCAATTGGTCGACCAAGC-5'

are synthesized, annealed, thereafter ligated together and digested with EcoRI and AsuII to obtain a combined DNA fragment of the two linkers. In the formulae set forth above, the DNA sequences corresponding to the tryptophan operator are underlined.

(2) Removal of the PvuI site in the ampicillin-resistance gene from plasmid pHSG666:

Bacterial cells, each carrying plasmid pHSG666, are treated with nitrosoguanidine according to the method described in "Experiments in Molecular Genetics" [Jeffrey Miller (ed.), pp. 125-129, Cold Spring Harbor Laboratory, U.S.A., (1972)] to prepare a plasmid DNA. The plasmid DNA is digested with PvuI and introduced into bacterial cells, which are then subjected to selection for ampicillin-resistance. This procedure is repeated several times to erase both of the two PvuI sites in the plasmid. It is then confirmed that the plasmid DNA obtained from this transformant is not digested by PvuI.

(3) Replacement of the promoter region of the streptomycin-sensitivity gene by the synthetic tryptophan promoter:

The promoter region of the streptomycin-sensitivity gene is removed from the plasmid obtained in the procedure (2) above by digestion with EcoRI and AsuII, and therein is inserted the synthetic DNA fragment obtained in the procedure (1), which fragment codes for the tryptophan promoter/operator and has cohesive ends for EcoRI and AsuII. Furthermore, in order to erase the EcoRI site located upstream of the streptomycin-sensitivity gene, this plasmid DNA is digested completely with EcoRI and partially with SspI, and thereafter the treatment with T4 DNA polymerase for blunt end generation is followed by religation. Plasmid DNAs are prepared from the resultant transformants, and the DNA sequence at the ligated junction is determined to select for DNAs lacking in CAAT between the EcoRI site and the SspI site located at the left end of the sequencing formulae given in the procedure (1) above. The streptomycin-sensitivity gene of this plasmid is still that of pHSG666 having a limited number of cloning sites. Therefore, in order to replace this by the streptomycin-sensitivity gene of pHSG670, a DNA fragment containing the tryptophan promoter region and the upstream region of the ampicillin-resistance gene of this plasmid is isolated by digestion with BglI and XhoI while a fragment containing a large portion of the streptomycin-sensitivity gene and the replication region of pHSG670 is separately isolated with the corresponding two enzymes, and both the fragments thus purified are finally religated together. The plasmid thus obtained was named pHSG672.

(4) Introduction of the amber mutation into the streptomycin-sensitivity gene:

The DNA portion between the SphI site and the MluI site of the streptomycin-sensitivity gene, coding for from the 26th amino acid, alanine, to the 37th amino acid, valine, counting from the first methionine residue of the gene, is replaced by the DNA linker having the following formula:

$$5'-CCCCTAGAAGCCTGGCATATGCACA-3'$$

$$3'-GTACGGGCATCTTCGCACCGTATACGTGTGCGC-5'$$

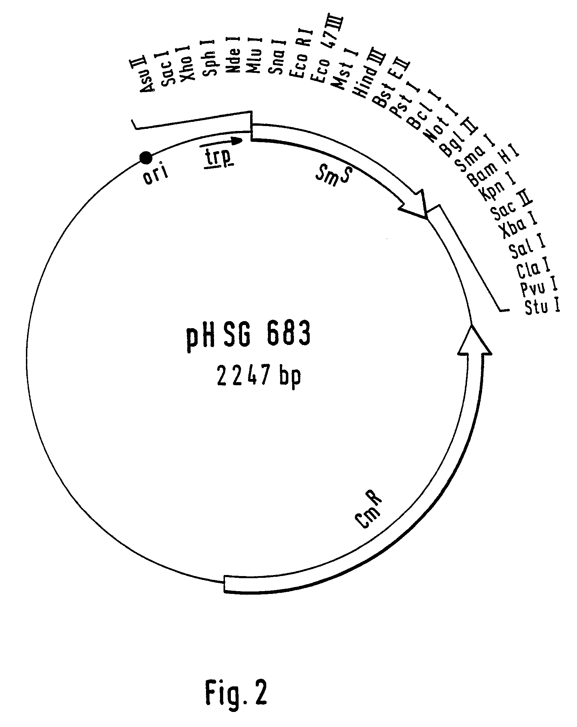

Consequently, the 29th codon CAG for glutamine residue is altered to the amber codon TAG, and the 32nd GGT codon for glycine residue and the 33rd codon CTG for valine residue are altered to the GGC codon for glycine and the ATA codon for isoleucine, respectively. As a result, CATATG, an NdeI site, is newly introduced. This plasmid is named pHSG673, and a restriction map thereof is shown in FIG. 2.

(5) Analysis of the modified streptomycin-sensitivity gene of plasmid pHSG673:

When the plasmid pHSG673 molecules are introduced into E. coli F⁻Z⁻4M15 recA (strA$^R$, sup$^+$), it is confirmed that the resultant transformants exhibit streptomycin resistance. Furthermore, plasmid DNAs are extracted from the transformants, and the cells of the HB101 strain (strA$^R$, supE$^-$) are transformed therewith to determine the ratio of the number of the cells with double transformation, i. e., ampicillin and streptomycin-resistances, to the number of the ampicillin-resistant transformants. The concentrations of ampicillin and streptomycin in the medium are 100 microgram/ml each. As a selective medium, LB broth ("Experiments in Molecular Genetics", loc. cit., p. 443) is used for pHSG664 carrying the wild-type streptomycin-sensitivity gene and for pHSG666 with limited alterations in the codons of the gene for wild-type amino acid sequences driven by the authentic streptomycin gene promoter and as well for pHSG670 with limited alterations in the codons and as well in the amino acid sequences driven by the authentic promoter. Furthermore, for pHSG672 in which the promoter has been replaced in pHSG670 by the tryptophan promoter and for pHSG673 in which the amber mutation has been introduced, CA medium [containing 10 g of Vitamin Assay Casamino Acids (Difco Laboratories), 1 g of glucose, 5 g of sodium chloride, 100 micrograms of thiamine and one litre of water] is used to deplete tryptophan from the medium. The results are shown in the following Table 2:

Table 2

| Plasmid | Medium | $Ap^R$, $Sm^R$ / $Ap^R$ |
|---------|--------|-------------------------|
| pHSG664 | LB | $8 \times 10^{-4}$ |
| pHSG666 | LB | $6 \times 10^{-3}$ |
| pHSG670 | LB | $1 \times 10^{-1}$ |
| pHSG672 | CA | $1 \times 10^{-3}$ |
| pHSG673 | CA | $2 \times 10^{-4}$ |
| pHSG673 | CA + IAA | $<2 \times 10^{-6}$ |

IAA in the table indicates that the medium contains 0.1 mM indoleacrylic acid.

From these results, it is evident that the appearing of background colonies is suppressed by two orders of magnitude in the case where the promoter is replaced by the tryptophan promoter and further by three orders of magnitude where the amber codon is introduced. Furthermore, by inducing treatment with indoleacrylic acid, the background colonies entirely disappear.

EP 0 309 961 A1

```
                    -35                    operator                                    (Met)Ala-Thr-
ATTCTGAAATGAGCTGttgacaATTAATCATcgAactagttaactagtAcgTTCACGTAAAAAGGGTATCGACC ATG GCA ACA
                                  Spe|  Hpa|  Spe|                                  Nco|

Val-Asn-Gln-Leu-Val-Arg-Lys-Pro-Arg-Ala-Arg-Lys-Val-Ala-Lys-Ser-Asn-Val-Pro-Ala-Leu-Glu-
GTT AAC CAG CTG GTT CGA AAG CCG CGA GCT CGT AAA GTG GCC AAA TCT AAC GTT CCG GCT CTC GAG
Hpa|     Pvu▯    Asu▯            Sac|            Bal|                                 Xho L

Ala-Cys-Pro-Gln-Lys-Arg-Gly-Ile-Cys-Thr-Arg-Val-Tyr-Thr-Thr-Thr-Pro-Lys-Lys-Pro-Asn-Ser-
GCA TGC CCG tag AAG CGT GGC ATA TGC ACA CGC GTA TAC ACT ACT ACT CCG AAG AAA CCG AAT TCA
Sph|                           Nde|     Mlu| Sna|                                    EcoR|

Ala-Leu-Arg-Lys-Leu-Cys-Arg-Val-Arg-Leu-Thr-Asn-Gly-Phe-Glu-Val-Thr-Ser-Tyr-Ile-Gly-Gly-
GCG CTG CGC AAG CTT TGC CGC GTA CGC CTG ACC AAC GGT TTC GAG GTC ACC TCA TAT ATA GGT GGT
Eco47▯  Mst| Hind▯                                        BstE▯

Glu-Gly-His-Asn-Leu-Gln-Glu-His-Ser-Val-Ile-Leu-Ile-Arg-Gly-Gly-Arg-Val-Lys-Asp-Leu-Pro-
GAA GGA CAC AAC CTG CAG GAA CAC TCT GTT ATC CTG ATC AGA GGC GGC CGC GTT AAA GAT CTG CCC
              Pst|                              Bcl|     Not| Xma▯           Bgl▯

Gly-Ile-Arg-Tyr-His-Thr-Val-Arg-Gly-Ala-Leu-Asp-Cys-Ser-Gly-Val-Lys-Asp-Arg-Arg-Gln-Asp-
GGG ATC CGG TAC CAC ACC GTC CGC GGC GCT CTA GAC TGC TCC GGA GTA AAG GAC CGT CGA CAG GAT
Sma| BamH| Kpn|            Sac▯    Xba|         Acc▯              Sal|

Arg-Ser-Lys-Tyr-Gly-Val-Lys-Arg-Pro-Lys-Ala trm
CGA TCG AAA TAC GGT GTA AAA CGT CCG AAG GCt taa tag AAGCT
Cla I. Pvu I                          StuI
```

# Table 1

## Claims

1. A DNA segment containing a streptomycin-sensitivity gene with a nonsense codon or nonsense codons in an essential area of the coding region.

2. A DNA segment as set forth in claim 1 wherein an inducible promoter is located upstream of said streptomycin-sensitivity gene so as to control the expression of said gene.

3. A DNA segment as set forth in claim 2 wherein the nonsense codon in the coding region is the amber codon.

4. A DNA segment as set forth in claim 2 wherein the inducible promoter is the tryptophan promoter.

5. A DNA segment as set forth in claim 2, which has the nucleotide sequence shown in Table 1, and in which the nonsense codon in the coding region is the amber codon and the inducible promoter is the tryptophan promoter.

6. A plasmid vector selected from the group consisting of a plasmid vector named pHSG673 and a plasmid vector named pHSG683, each of which carries a DNA sequence having a streptomycin-sensitivity gene with a nonsense codon in the coding region and an inducible promoter upstream thereof so as to control the expression of said gene.

7. A microorganism containing a plasmid vector selected from the group consisting of a plasmid vector named pHSG673 and a plasmid vector named pHSG683, each of which carries a DNA sequence containing a streptomycin-sensitivity gene with a nonsense codon in the coding region and an inducible promoter upstream thereof so as to control the expression of said gene.

8. A microorganism as set forth in claim 7 wherein the microorganism is Escherichia coli.

Claims for the following Contracting State: ES

1. A process for the selection of microorganisms which have been transformed with a plasmid containing a streptomycin-sensitivity gene, characterized in that the streptomycin-sensitivity gene contains one or more nonsense codons in an essential area of its coding region.

2. A process according to claim 1, characterized in that the streptomycin-sensitivity gene is controlled by an inducible promoter.

3. A process according to claim 2, characterized in that the promoter is the tryptophan promoter.

4. A process according to one or more of the preceding claims, characterized in that the nonsense codon is the amber codon.

5. A process according to one or more of the preceding claims, characterized in that the DNA region comprising the streptomycin-sensitivity gene has the DNA sequence of Table 1.

6. A process according to one or more of the preceding claims, characterized in that the plasmid containing the streptomycin-sensitivity gene is the plasmid pHSG 673 of Fig. 1 or the plasmid pHSG 683 of Fig. 2.

7. A process according to one or more of the preceding claims, characterized in that the microorganism is a bacterium.

8. A process according to claim 7, characterized in that the bacterium is E. coli.

Fig.1

Fig. 2

| DOCUMENTS CONSIDERED TO BE RELEVANT | | | EP 88115803.4 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | EP - A1 - 0 223 156 (HOECHST JAPAN LIMITED)<br><br>* Claims 1,6,9 *<br><br>-- | 1,6,7 | C 12 N 15/00<br>C 07 H 21/04<br>C 12 N 1/20<br>(C 12 N 1/20;<br>C 12 R 1:19) |
| D,A | GENE, vol. 41, 1986, Amsterdam<br>T.HASHIMOTO-GOTOH et al. "Improved vector, pHSG 664, for direct streptomycin-resistance selection: cDNA cloning with G:C-tailing procedure and subcloning of double-digest DNA fragments."<br>pages 125-128<br><br>* Totality *<br><br>---- | 1,6 | |

| | TECHNICAL FIELDS SEARCHED (Int. Cl.4) |
|---|---|
| | C 12 N<br>C 07 H |

The present search report has been drawn up for all claims

| Place of search | Date of completion of the search | Examiner |
|---|---|---|
| VIENNA | 20-12-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82